(19) Europäisches Patentamt
European Patent Office
Office européen des brevets

(11) **EP 1 010 421 A1**

(12) **EUROPÄISCHE PATENTANMELDUNG**

(43) Veröffentlichungstag:
21.06.2000 Patentblatt 2000/25

(51) Int. Cl.⁷: **A61K 7/50**, C11D 1/83,
A61K 7/48, C11D 1/66,
A61K 7/06

(21) Anmeldenummer: 99124450.0

(22) Anmeldetag: 08.12.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.12.1998 DE 19858241**

(71) Anmelder:
**Henkel Kommanditgesellschaft auf Aktien**
**40589 Düsseldorf-Holthausen (DE)**

(72) Erfinder:
• **Schelges, Heike**
**47807 Krefeld (DE)**
• **Scholz, Wolfhard**
**47829 Krefeld (DE)**

(54) **Verdickte Zubereitung wasserlöslicher Tenside**

(57) Wäßrige tensidhaltige Zubereitungen, die eine Verdickungsmittel-Kombination aus einem Magnesium-aluminiumsilikat, einem organischen Verdickungsmittel ausgewählt aus der Gruppe der nicht-ionogenen Polysaccharide und Polysaccharid-Derivate und einem Fettalkoholethoxylat enthalten, zeichnen sich durch eine verbesserte Konsistenz, Lagerstabilität und Cremigkeit des Schaums aus. Die erwünschte Viskosität der erfindungsgemäßen Zubereitungen läßt sich mit Hilfe des Fettalkoholethoxylats besonders leicht einstellen.

EP 1 010 421 A1

Printed by Xerox (UK) Business Services
2.16.7 (HRS)/3.6

**Beschreibung**

Die Erfindung betrifft wäßrige tensidhaltige Zubereitungen mit einem speziellen Verdickersystem, welches die Herstellung, Viskositätseinstellung und anwendungs-technischen Eigenschaften kosmetischer Rezepturen, insbesondere die Cremigkeit, das Schaumverhalten und die Verteilbarkeit deutlich verbessert.

Körperreinigungsmittel auf wäßriger Basis, beispielsweise Shampoos, Duschbäder, Dusch- und Badeöle sowie Duschgele und Gesichtsreinigungsprodukte enthalten zur Stabilisierung und Konsistenzregulierung Verdickungsmittel (Quellungsmittel). Aufgabe der Verdickungsmittel ist es, die Viskosität der Formulierungen zu erhöhen und im gewünschten Bereich zu regulieren, so daß die Produkte für den Verbraucher leicht handhabbar sind, d. h. beim Auftragen nicht zu schnell zerfließen. Gleichzeitig sollten die Verdickungsmittel leicht verarbeitbar sein und die Eigenschaften anderer Wirkstoffe nicht negativ beeinflussen.

Verdickungsmittel sind dem Fachmann in großer Zahl aus der einschlägigen Literatur bekannt und im Handel erhältlich. Der Begriff Verdickungsmittel wird synonym zu Stabilisator, Konsistenzregulator, Binde- oder Quellmittel verwendet. Verdickungsmittel lassen sich in anorganische und organische Verdickungsmittel unterteilen. Zu den anorganischen Verdickungsmitteln zählen beispielsweise feinteilige Kieselsäuren und Schichtsilikate sowie bestimmte anorganische wasserlösliche Salze. Die organischen Verdickungsmittel werden üblicherweise unterteilt in natürliche (z. B. Agar-Agar, Alginate, Carrageen, Tragant, Gummi arabicum, Pektine, Polyosen, Guar-Mehl, Johannisbrotbaumkernmehl, Stärke, Dextrine, Gelatine, Casein) und vollsynthetische Verdickungsmittel (Acryl- und Methacrylsäurepolymere, Vinylpolymere, Polycarbonsäuren, Polyether, Polyimine, Polyamide) sowie abgewandelte Naturstoffe (z. B. Celluloseether und deren Derivate, Fettalkoholalkoxylate, alkoxylierte Glucoseester, alkoxylierte Glycerid-Derivate). Verdickungsmittel finden Einsatz in technischen, kosmetischen und pharmazeutischen Präparaten und sind in vielen Lebensmitteln enthalten.

Zur Herstellung von Körperreinigungsprodukten werden häufig vernetzte Acrylsäurepolymerisate, die beispielsweise unter dem Handelsnamen Carbopol® bekannt sind, als Verdickungsmittel verwendet. Aufgrund der Agglomerisationsneigung mit Tensiden, insbesondere mit anionischen Tensiden, sind diese in bestimmten Rezepturen nur bedingt und unter hohem technischen Aufwand (lange Homogenisierungszeiten) großtechnisch einsetzbar. Formulierungen mit Acrylsäurepolymerisaten zeigen weiterhin deutlich schlechtere Schaumeigenschaften und hinterlassen beim Anwender ein typisches, oft als unangenehm empfundenes Hautgefühl.

Bedingt durch viskositätsrelevante Rohstoffschwankungen sind die rheologischen Eigenschaften von Körperreinigungsprodukten üblicherweise nicht exakt reproduzierbar, was sich nachteilig für die Produktion auswirken kann.

Aufgabe war es daher, wäßrige tensidhaltige Zubereitungen mit Verdickungsmitteln zu entwickeln, die im Vergleich zu acrylsäurepolymerisathaltigen Formulierungen keine Neigung zur Agglomerisation zeigen, über verbesserte Schaumeigenschaften verfügen und beim Anwender ein verbessertes Hautgefühl hinterlassen . Die Zubereitungen müssen weiterhin eine gute Lagerstabilität und das gewünschte Fließverhalten aufweisen. Die Verdickungsmittel-Kombination soll hierbei eine in der Technik leicht umsetzbare Konsistenzregulierung ermöglichen.

Hautpflegemittel, die als Verdickungsmittel u. a. die Kombination Acrylsäurepolymerisat®/Fettalkoholethoxylat oder Magnesiumaluminiumsilikat/ionogenes Polysaccharid enthalten, sind in EP 0 238 302, EP 0 824 086 und DE 3779346 beschrieben. Bezüglich ihrer Verträglichkeit mit weiteren Rohstoffen, insbesondere mit Tensiden, und ihrer anwendungstechnischen Eigenschaften können derartige Kombinationen jedoch nicht völlig befriedigen. Körperreinigungsmittel, die zur Konsistenzregulierung u. a. die Kombination Magnesiumaluminiumsilkat/Methylhydroxypropylcellulose enthalten, sind in EP 0 339 539 beschrieben. Eine Feineinstellung der Viskosität im gewünschten Bereich ist mit dieser Verdickungsmittelkombination jedoch nicht möglich. Eine Tensid-Kombination, die ebenfalls Magnesiumaluminiumsilkat/Methylhydroxypropylcellulose als Verdickungsmittel enthält, ist in US 5 696 069 beschrieben. Bezüglich des Schaumverhaltens und der Pflegewirkung kann jedoch auch diese Kombination nicht völlig befriedigen.

Überraschenderweise wurde gefunden, daß wäßrige Zubereitungen die als Tenside eine Kombination aus anionischem Tensid und Alkylpolyglykosid und als Verdickungsmittel eine Kombination aus Magnesiumaluminiumsilicat, wasserlöslichen, nicht-ionogenen organischen Polymeren und Fettalkoholethoxylaten enthalten, sich nicht nur durch deutlich besseres Schaumverhalten ohne Änderung der Tensidkomponente(n) oder des Tensidgehalts, sondern auch durch eine erhöhte Pflegeleistung ohne Zusatz weiterer Pflegekomponenten auszeichnen, d. h. beim Anwender ein angenehmeres Hautgefühl erzeugen. Das Dreikomponenten-Verdickersystem ermöglicht außerdem eine besonders einfache Einstellung der Viskosität im gewünschten Bereich.

Gegenstand der Erfindung sind daher wäßrige tensidhaltige Zubereitungen, dadurch gekennzeichnet, daß als Tenside eine Kombination aus a) mindestens einem anionischen Tensid und b) mindestens einem Alkylpolyglycosid und als Verdickungsmittel eine Kombination aus (c) einem Magnesiumaluminiumsilikat, (d) einem wasserlöslichen, nicht-ionogenen organischen Polymer und (e) einem Fettalkoholethoxylat enthalten ist.

Wäßrige tensidhaltige Zubereitungen im Sinne der Erfindung können ca. 10-90 Gew.%, vorzugsweise 40-80 Gew.%, Wasser und ca. 1-50 Gew.-% Tenside, vorzugsweise 5-20% Tenside, enthalten.

Anionische Tenside sind dem Fachmann in großer Zahl als besonders schaumstarke Tenside aus einschlägigen Handbüchern bekannt und im Handel erhältlich. Die erfindungsgemäßen Zubereitungen enthalten als obligatorische Tensidkomponente anionische Tenside, beispielsweise Alkylsulfate, Alkylethersulfate, Sulfosuccinate und deren Derivate, Alkylethercarboxylate, Acylisethionate, Acylsarkosinate und Acyltaurine mit linearen Alkyl- oder Acylgruppen (12 - 18 C-Atome) in Form ihrer Alkali-, Magnesium-, Ammonium- oder Alkanolammoniumsalze enthalten. Die anionischen Tenside können dabei in einer Menge von 1 - 30 Gew.-% in den erfindungsgemäßen Zusammensetzungen enthalten sein.

Bevorzugt geeignete Zubereitungen sind dadurch gekennzeichnet, daß ein anionisches Tensid vom Typ der Alkylethersulfate enthalten ist. Alkylethersulfate der Formel $R^1O(C_2H_4O)_m\,SO_3^{(-)}M^{(+)}$, in der $R^1$ eine Alkyl- oder Alkenylgruppe mit 12 - 18 C-Atomen, m = 1 - 4 und $M^{(+)}$ ein Alkali-, Magnesium-, Ammonium- oder Alkanolammoniumion ist, sind besonders geeignet. Sie sind bevorzugt in einer Menge von wenigstens 5 Gew.-% der gesamten Zusammensetzung enthalten.

Wegen ihrer hervorragenden biologischen Abbaubarkeit, ihrer synergistischen Schaumwirkung mit Ethersulfaten und ihres Pflegepotentials sind Alkylpolyglycoside (APG) als weitere obligatorische Tensidkomponente in den erfindungsgemäßen Zubereitungen enthalten. Alkylpolyglycoside lassen sich durch Umsetzung von Glucose oder von Oligosacchariden mit primären Alkoholen herstellen, wobei meist Alkohole mit 8 bis 22 C-Atomen eingesetzt werden. Man erhält sie auch durch Umacetalisierung von Stärke mit z. B. niederen Alkoholen und erneute Umacetalisierung mit einem $C_8$-$C_{22}$-Fettalkohol.

Als Glykosidreste eignen sich sowohl Monoglykoside als auch oligomere Glykoside mit einem Oligomerisationsgrad bis etwa 3. Geeignete Alkylglykoside für die Herstellung der erfindungsgemäßen Zubereitungen lassen sich durch die allgemeine Formel $R^2O$ - $(G)_x$ beschreiben, worin $R^2$ einen aliphatischen Rest eines primären Fettalkohols mit 8 bis 22 C-Atomen und $(G)_x$ ein Oligoglucosidrest mit einem mittleren Oligomerisationsgrad x von 1 bis 3 darstellt. Der mittlere Oligomerisationsgrad ergibt sich aus den molaren Anteilen der einzelnen Oligomeren durch Division der Summe der Struktureinheiten durch die Summe der Moleküle (vgl. Principles of Polymer Chemistry, Paul J. Flory, Cornell University Press, Ithaca, New York 1953, Seite 35 - 36). Bevorzugt sind Oligoglucoside nach obiger Formel, worin $R^2$ einer lineare Alkylgruppe mit 8 bis 16 C-Atomen und $(G)_x$ einem Oligoglucosidrest mit einem mittleren Oligomerisationsgrad x von 1 bis 2 entspricht. Solche Produkte sind z. B. unter dem Warenzeichen Plantaren® oder Plantacare® im Handel.

Die erfindungsgemäßen Zubereitungen können ca. 1-15 Gew.-% Alkypolyglycoside enthalten, wobei ein Bereich von 2-5 Gew.-% bevorzugt wird.

Dem Fachmann sind zahlreiche weitere nichtionische Tenside bekannt. Die erfindungsgemäßen Zubereitungen können z. B. weiterhin nichtionische Tenside aus den Verbindungsklassen der Fettalkoholethoxylate und -propoxylate, Fettsäuremonoethanolamine und der alkxoxylierte Alkylpolyglycoside enthalten.

Die erfindungsgemäßen Zubereitungen enthalten Magnesiumaluminiumsilikate als obligatorische Komponente der Verdickungsmittel-Kombination. Magnesiumaluminiumsilikate zählen zu den Tonmineralien mit Blattstruktur. Deren Quellvermögen ist auf den Gehalt an sogenannten Phyllo- oder Schichtsilikaten zurückzuführen, die Wassermoleküle zwischen den Schichten einlagern können. Dies führt zu einer Vergrößerung der Schichtabstände, was als Quellung des Materials wahrgenommen wird. Wegen seiner leichten Verfügbarkeit und seinen guten anwendungstechnischen Eigenschaften wird Magnesiumaluminiumsilkat als Verdickungsmittel in Kosmetika eingesetzt.

Die erfindungsgemäßen tensidhaltigen Zubereitungen enthalten Magnesiumaluminiumsilikat in Mengen von 0.1-5 Gew.-%, bevorzugt jedoch in 0.5-1.5 Gew.-%. Tensidhaltige Zubereitungen, die dadurch gekennzeichnet sind, daß ein *saures* Magnesiumaluminiumsilikat, insbesondere vom Typ Veegum® Ultra (Vanderbilt) enthalten ist, sind bevorzugt. Als sauer werden solche Magnesiumaluminiumsilikate bezeichnet, die in einer 3-7%igen (Gew.-%) Dispersion in Wasser pH-Werte von 3.0 bis 6.0 aufweisen.

Als weitere anorganische Verdickungsmittel können feinteilige Kieselsäuren, andere Schichtsilikate und Tonmaterialien sowie diverse wasserlösliche anorganische Elektrolytsalze in den erfindungsgemäßen Zubereitungen enthalten sein.

Als zweite obligatorische Komponente enthält die erfindungsgemäße Verdickungsmittel-Kombination ein wasserlösliches, nicht-ionogenes organisches Polymer. Hierbei kann es sich um ein synthetisches oder natürliches Polymer handeln. Als wasserlöslich im Sinne der Erfindung werden Polymere bezeichnet, die sich bei 20°C in einer Menge von wenigstens 1 Gew.-% in Wasser lösen.

Zu den wasserlöslichen, nicht-ionogenen synthetischen Polymeren, die in der erfindungsgemäßen Verdickungsmittel-Kombination enthalten sein können, zählen beispielsweise Polyvinylpyrrolidin, Polyvinylalkohol und Polyethylenoxide, wobei insbesondere Polymere mit einem Molekulargewicht von 500000 bis 5000000 zur Verdickung geeignet sind.

Wäßrige tensidhaltige Zubereitungen, die dadurch gekennzeichnet sind, daß ein wasserlösliches, nichtionogenes organisches Polymer, ausgewählt aus der Gruppe der natürlichen Polymere, enthalten ist, sind bevorzugt geeignet.

Zu den wasserlöslichen, nicht-ionogenen natürlichen Polymeren zählen u. a. Polysaccharide oder Polysaccharid-Derivate. Sie sind nicht nur mit anionsichen Tensiden gut kombinierbar, sondern weisen mit Veegum® einen synergistischen Verdickungseffekt auf (Datenblätter von Vanderbilt). Bevorzugt geeignet sind daher Zubereitungen, die dadurch gekennzeichnet sind, daß das wasserlösliche, nicht-ionogene organische Polymer ausgewählt ist aus der Gruppe der nicht-ionogenen Polysaccharide oder Polysaccharid-Derivate. Hierbei handelt es sich um Homo- und Heteropolysaccharide, die als Zuckerkomponente Glucose, Fructose, Mannose, Galactose, Talose, Gullose, Allose, Idose, Arabinose, Xylose, Lyxose oder Ribose enthalten. Bevorzugt enthalten sind die üblichen, in der Kosmetik verwendeten wasserlöslichen Polysaccharide oder die wasserlöslichen nicht-ionogenen Derivate wasserunlöslicher Polysaccharide. Erfindungsgemäß geeignete organische Verdickungsmittel sind u. a. lösliche Stärke, deren Veretherungsprodukte sowie die wasserlöslichen Celluloseether wie z. B. Methylcellulose, Hydroxyethylcellulose, Hydroxypropylcellulose und Methylhydroxypropylcellulose. Die erfindungsgemäßen Zubereitungen enthalten üblicherweise 0.1-4.0 Gew.-%, bevorzugt 0.2-2.0 Gew.-% der Polysaccharide.

Methylhydroxypropylcellulosen sind in den erfindungsgemäßen Zubereitungen besonders gut mit Tensiden kombinierbar, da sie nicht zur Agglomerisierung neigen und die Zubereitungen leicht homogeniesierbar sind. Bevorzugt sind daher Zubereitungen, die dadurch gekennzeichnet sind, daß das nicht-ionogene Polysaccharid-Derivat eine Methylhydroxypropylcellulose ist. Insbesondere sind niedermolekulare Methylhydroxypropylcellulosen mit einer Viskosität von 3-6 Pa•s (2 Gew.-% in Wasser, 20°C), wie beispielsweise Methocel® E4M prem., geeignet.

Fettalkoholethoxylate sind als dritte obligatorische Verdickungskomponente in den erfindungsgemäßen Zubereitungen enthalten. Zubereitungen, die dadurch gekennzeichnet sind, daß ein Fettalkoholethoxylat mit einem HLB-Wert von 3-9 enthalten ist, sind im Sinne der Erfindung bevorzugt, da sie in Wasser nur wenig löslich sind, jedoch in Lösungen wasserlöslicher Tenside solubilisiert werden und dabei eine Verdickung dieser Lösungen bewirken. In den erfindungsgemäßen Zubereitungen sind sie in Mengen von 0.1-3.0 Gew.-%, bevorzugt 0.2-1.0 Gew.-% enthalten. Der HLB-Wert des Fettalkoholethoxylats wird hier definiert als $HLB = 0.2 \cdot (100\% - L)$, wobei L der lipophile Anteil, d.h. der Fettalkylrest, des Fettalkoholethoxylats in Gew.-% ist. Geeignet sind z. B. Fettalkoholethoxylate, die durch Anlagerung von 1-4 Mol Ethylenoxid an gesättigte oder ungsättigte lineare Fettalkohole mit 12-22 C-Atomen erhältlich sind. Insbesondere geeignet sind Fettalkoholethoxylate mit einem mittleren Ethoxylierungsgrad von 2-3, die sich aus Fettalkoholen mit 12-14 Kohlenstoffatomen ableiten. Als mittleren Ethoxylierungsgrad bezeichnet man den Quotienten der eingesetzten Mole Etylenoxid pro Mol Fettalkohol.

Dem Fachmann ist bekannt, daß bei der katalytischen Umsetzung eines Fettalkohols mit Ethylenoxid ein Polyglykolether-Gemisch unterschiedlich hoch ethoxylierter Homologer entsteht, deren Verteilung in Abhängigkeit des Katalysators u. der Ethylenoxid-Menge zwischen einer der Statistik entsprechenden Gauss- u. einer unselektiven Schulz-Flory-Kurve variieren kann. So wird z. B. in Gegenwart von Alkalimetallen, Alakimetallhydroxiden oder Alkalimetallalkoholaten eine breite, unter Verwendung von Erdalkali-Salzen, Hydrotalciten, Ethercarbonsäuren, Erdalkalimetalloxiden, -hydroxiden oder -alkoholaten dagegen eine eingeengte (narrow-range) Homologen-Verteilung erhalten. Bevorzugt geeignet sind wäßrige tensidhaltige Zubereitungen, die dadurch gekennzeichnet sind, daß die ethoxylierten Fettalkohole eine eingeengte Homologenverteilung aufweisen, wobei mindestens 50 Gew.-% des Homologengemisches aus Homologen mit x ± 1 Glycolethergruppen besteht (x = angelagerte Molmenge Ethylenoxid), und der Gehalt an freiem Fettalkohol unter 12 Gew.-% liegt. Ein derartiges Fettalkoholethoxylat-Gemisch ist beispielsweise unter dem Warenzeichen Arlypon® F im Handel.

Als weitere organische Verdickungsmittel können u. a. Fettsäurealkanolamide, wie Kokosfettsäuremonoethanolamid, Laurinsäuremonoethanolamid, Ölsäurediethanolamid und Kokosfettsäurediethanolamid sowie Polyethylenglykoldifettsäureester enthalten sein.

Eine bevorzugte Variante der erfindungsgemäßen Zubereitungen ist dadurch gekennzeichnet, daß zusätzlich mindestens ein wasserlösliches Tensid ausgewählt aus der Gruppe der zwitterionischen, amphoteren und kationischen Tenside enthalten ist.

Zwitterionische Tenside sind ebenfalls seit langem bekannt und werden in einer Vielzahl kosmetischer Rezepturen eingesetzt. Als zwitterionische Tenside werden solche oberflächenaktiven Verbindungen bezeichnet, die im Molekül mindestens eine quartäre Ammoniumgruppe und mindestens eine -COO$^{(-)}$ - oder -SO$_3^{(-)}$-Gruppe tragen. Die bekannteste und am weitesten verbreitete Gruppe dieser Tenside ist die der Betain-Tenside, bei denen die quartäre Ammoniumgruppe zwei Methylsubstituenten trägt. Bevorzugt sind daher wäßrige Zubereitungen, die dadurch gekennzeichnet sind, daß zusätzlich ein zwitterionisches Tensid vom Typ der Betain-Tenside enthalten ist. Geeignete zwitterionische Betain-Tenside sind u.a. N-Alkyl-N,N-dimethylammoniumglycinate, beispielsweise Kokosalkyldimethylammoniumglycinate, N-Acyl-aminopropyl-N,N-dimethylammoniumglycinate, beispielsweise Kokosacylaminopropyldimethylammoniumglycinate, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Als bevorzugte zwitterionische Betain-Tenside können in den erfindungsgemäßen wäßrigen Zubereitungen Kokosacylaminopropyldimethylammoniumglycinate, die unter der INCI-Bezeichnung Cocamidopropylbetain bekannt sind, enthalten sein, wobei Cocoamidopropylbetain, das von $C_8$ - $C_{18}$—Kokos- oder Palmkernfettsäuren abgeleitet ist, besonders geeignet ist. Solche Produkte sind z. B. unter dem Warenzeichen Dehyton[®]K im Handel. In den erfindungsgemäßen Zubereitungen können ca. 0.5-10 Gew.-% zwitterionische Tenside, bevorzugt ca. 0.5-5.0 Gew.-%, enthalten sein.

Auch Amphotenside können in den erfindungsgemäßen Zubereitungen enthalten sein. Unter ampholytischen Tensiden werden solche oberflächenaktiven Verbindungen verstanden, die außer einer $C_8$-$C_{18}$-Alkyl- oder Acylgruppe im Molekül mindestens eine freie Aminogruppe und mindestens eine -COOH- oder -$SO_3$H-Gruppe enthalten. Sie haben die Eigenschaft, in saurer Lösung durch Protonierung am tertiären Stickstoffatom wie Kationtenside und im alkalischen Bereich durch Salzbildung an der Carboxylgruppe wie anionische Tenside zu reagieren. Amphotenside können innere Salze ausbilden. Beispiele für geeignete Amphotenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte Amphotenside sind das N-Kokosalkylaminopropionat, das Kokosacylaminoethylaminopropionat, $C_{12-18}$-Acylsarcosine und Cocoamphoglycinate. Zur letzten Gruppe gehört beispielsweise das unter dem Warenzeichen Dehyton[®]G im Handel erhältliche Produkt.

Als kationische Tensid-Zusätze zu den erfindungsgemäßen Zubereitungen kommen vor allem quartäre Ammoniumverbindungen in Frage. Geeignet sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex[®] vertriebenen Methylhydroxyalkyldiacyloxyalkylammonium-methosulfate und die entsprechenden Produkte der Dehyquart[®]-Reihe als kationische Tenside eingesetzt werden. Weitere erfindungsgemäß verwendbare kationische Tenside stellen die quaternisierten Proteinhydrolysate dar.

Als Tensidkombination ist in den erfindungsgemäßen Zubereitungen vor allem die Kombination aus anionischen, nichtionischen (APG) und zwitterionischen Tensiden bevorzugt. Um ein besonders gutes Schaumverhalten mit der gewünschten Feinblasigkeit und Cremigkeit des Schaums zu erzielen, sind die bevorzugten Mengenverhältnisse ca. 10:(1-6):(0.5-2) Gew.-% (anionisch:nichtionisch:zwitterionisch).

Geeignete weitere Zusätze in den erfindungsgemäßen wäßrigen tensidhaltigen Zubereitungen sind Avivagemittel, perlglanzbildende Stoffe, Farbstoffe, Duftstoffe und dafür geeignete Emulgatoren, wasserlösliche Polyole wie z. B. Glycerin, Sorbit, Propylenglycol oder Polyethylenglycol, Elektrolytsalze, pH-Stellmittel und kosmetische oder dermatologische Wirkstoffe sowie Wachs- und Ölkomponenten und Rückfettungsmittel. Unter den Ölen sind pflanzliche Öle und Silikonöle wegen ihrer guten Hautveträglichkeit bevorzugt.

Der pH-Wert der erfindungsgemäßen Zubereitungen liegt üblicherweise bei 4-8, wobei ein Bereich von 4-6 bevorzugt ist. Zur Einstellung dieses pH-Wertes kann praktisch jede für kosmetische Zwecke verwendbare Säure oder Base eingesetzt werden. Für den Fall, daß zur pH-Wert-Einstellung eine Säure verwendet wird, kann es bevorzugt sein, eine Säure aus der Gruppe der Genußsäuren wie beispielsweise Essigsäure, Milchsäure, Weinsäure, Zitronensäure, Äpfelsäure, Adipinsaüre, Fumarsäure, Ascorbinsäure und Gluconsäure zu verwenden. Im Rahmen der Erfindung ist die Verwendung von Zitronensäure besonders bevorzugt.

Ebenfalls Gegenstand der Erfindung ist die Verwendung der erfindungsgemäßen wäßrigen tensidhaltigen Zubereitung zur Reinigung der Haut und Haare. Sie kann u. a. in Form von Shampoos, Duschgelen, Badezusätzen, Reinigungslotionen und -cremes verwendet werden. Für die erfindungsgemäßen Zubereitungen ist ein Viskosität von mindestens 1 Pa • s (Brookfield RVF, Helipath Spindel TC, 20 ° C) erforderlich, wobei ein Viskositätsbereich von ca. 5-50 Pa • s bevorzugt ist.

Gegenstand der Erfindung ist auch ein Verfahren zur Herstellung tensidhaltiger wäßriger Zubereitungen, dadurch gekennzeichnet, daß die Zugabe der Verdickungsmittel-Kombination in zwei Schritten erfolgt, wobei im ersten Schritt ein Magnesiumaluminiumsilikat und das Polysaccharid in Wasser vorgequollen und zugesetzt werden, und im zweiten Schritt ein Fettalkoholethoxylat zur Feineinstellung, d. h. zur Verdickung bis zur gewünschten Viskosität, zugesetzt wird. Rohstoffabhängige Schwankungen, die eine Viskositätseinstellung im gewünschten Bereich oft erschweren, können auf diese Weise problemlos behoben werden.

Die folgenden Beispiele sollen den Erfindungsgegenstand näher erläutern.

## Beispiele für Formulierungen

Alle Mengenangaben in nachfolgenden Tabellen beziehen sich auf Gew.-% Aktivsubstanz (AS).

Grundlage für alle Viskositätsmessungen:
Brookfield RVF, Helipath Spindel TC, 20 ° C

**Beispiel 1 (erfindungsgemäß)**

Zubereitung mit einer erfindungsgemäßen Verdickungsmittel-Kombination A/B/C enthaltend Veegum® Ultra (A), Methocel E4M prem. (B) und Arlypon F (C).

| Rohstoff | Gewichts % AS |
|---|---|
| Veegum® Ultra | 0,8 % |
| Methocel® E4M prem. | 0,6 % |
| Arlypon® F | 0,6 % |
| | |
| Texapon® N70 | 10,7% |
| Cocamidopropylbetain 45 % | 1,0% |
| Plantacare® 1200 UP | 4% |
| Lamesoft® PW 45 | 4,5 % |
| Citronensäure | 0,2 % |
| Na -Benzoat | 0,4 % |
| Wasser | ad 100 % |
| | |
| Viskosität 20 ° C | 30.000 mPa • s |
| Stabilität | Gut |
| Anwendungsverhalten | Sehr gute Verteilbarkeit; Erhöhte Pflegewirkung; verbessertes Schaumverhalten |

**Beispiel 2: Vergleichsbeispiele V1-V3 (nicht erfindungsgemäß)**

Deutlich schlechtere Eigenschaften ergeben sich für Zubereitungen, die nicht die Verdickungsmittel-Dreierkombination A/B/C enthalten, sondern beispielsweise nur eine oder zwei Komponenten davon. Für **V1** wurde Arlypon® F verwendet, für **V2** die Kombination Arlypon® F/Methocel® E4M prem., für **V3** die Kombination Veegum® Ultra/Arlypon® F.

| Rohstoff | % AS **V1** | % AS **V2** | %AS **V3** |
|---|---|---|---|
| Veegum® Ultra | | | 1,3 % |
| Methocel® E4M prem. | | 0,8 % | |
| Arlypon® F | 1,5 % | 0,6 % | 0,6 % |
| | | | |
| Texapon® N70 | 10,7% | 10,7 % | 10,7 % |
| Cocamidopropylbetain | 1,0 % | 1,0% | 1,0 |
| Plantacare® 1200 UP | 4,0 % | 4,0 % | 4,0 % |
| Lamesoft® PW 45 | 4,5% | 4,5% | 4,5% |
| Citronensäure | 0,2 % | 0,2 % | 0,2 % |
| Na -Benzoat | 0,4 % | 0,4 % | 0,4 % |

(fortgesetzt)

| Rohstoff | % AS V1 | % AS V2 | %AS V3 |
|---|---|---|---|
| Wasser | ad 100 % | ad 100 % | ad 100 % |
| | | | |
| Viskosität 20 ° C | 18.500 mPa•s | 30.000 mPa•s | 7500 mPa•s |
| Stabilität | Beginnende Trennung nach 4 Tagen bei 40 ° C | gut | Beginnende Trennung nach 4 Tagen bei 40 ° C |
| Anwendungsverhalten | Weniger cremig als Kombination A/B/C | Verteilbarkeit und Schaumverhalten schlechter als Kombination A/B/C | Deutlich zu dünn,schäumt schlechter und leerer als Kombination A/B/C |

**Beispiel 3: Vergleichsbeispiele V4-V6 (nicht erfindungsgemäß)**

Ebenso resultieren schlechtere Eigenschaften, wenn eine Komponente der erfindungsgemäßen Verdikkungsmittel-Kombination A/B/C ausgetauscht wird: in **V4** wurde die Kombination Hectabrite[®] AW/Methocel[®] E4M prem./Arlypon[®] F, in **V5** die Kombination Veegum[®] Ultra/Keltrol[®] F/ Arlypon[®] F und in **V6** die Kombination Laponite[®] XLS/ Methocel[®] E4M prem./Arlypon[®] F getestet.

| Rohstoff | % AS V4 | % AS V5 | % AS V6 |
|---|---|---|---|
| Laponite[®] XLS | | | 0,4 % |
| Hectabrite[®] AW | 1,6% | | |
| Veegum[®] Ultra | | 1 % | |
| Keltrol[®] F | | 1 % | |
| Methocel[®] E4M prem. | 0.4 % | | 0,6 % |
| Arlypon[®] F | 0,2% | 0,2 % | 0.2% |
| | | | |
| Texapon[®] N70 | 10,7 % | 10,7 % | 10,7 % |
| Cocamidopropylbetain | 1,0 % | 1,0 % | 1,0 % |
| Plantacare[®] 1200 UP | 4 % | 4 % | 4 % |
| Lamesoft[®] PW 45 | 4,5 % | 4,5 % | 4,5 % |
| Citronensäure | 0,2 % | 0,2 % | 0,2 % |
| Na -Benzoat | 0,4 % | 0,4 % | 0,4 % |
| Wasser | ad 100 % | ad 100 % | ad 100 % |
| | | | |
| Viskosität 20 ° C | 17.500 mPa•s | 20.000 mPa•s | 17000 mPa•s |
| Stabilität | Hectabrite[®] setzt beim Schleudern ab | Gut | Gut |
| Anwendungsverhalten | Gut | Schlechteres Schaumverhalten als A/B/C Kombination | Schlechteres Schaumverhalten als A/B/C Kombination |

# Anhang

## Verzeichnis der Warenzeichen der verwendeten Rohstoffe

1) Veegum® Ultra:

Saures Magnesiumaluminiumsilikat

Hersteller: R. T. Vanderbilt Company, Inc.

2) Methocel® E4M prem.

Methylhydroxypropylcellulose

Hersteller: Dow Chemical (Colorcon Ltd.)

3) Texapon® N70

$C_{12}$-$C_{14}$-Fettalkohol + 2EO-sulfat- Natriumsalz; ca. 70% Aktivsubstanz

Hersteller: Henkel France S.A., Nemours

4) Plantacare® 1200 UP

$C_{12}$-$C_{16}$-Alkyl-1.4-Glucosid; ca. 50% Aktivsubstanz, 47-50% Wasser

Hersteller: Henkel France S.A., Reims

5) Lamesoft® PW 45

Rückfettungsmittel, enthaltend Cetylpalmitat, Beheneth-10, hydriertes Rizinusöl, Glycerinstearat, Ameisensäure und ca. 52-57% Wasser

Hersteller: Henkel KGaA, Düsseldorf

6) Arlypon® F

$C_{12}$-$C_{14}$-Fettalkohol+2.5 EO; ca. 90% Aktivsubstanz, ca. 10% Wasser

Hersteller: Henkel KGaA, Düsseldorf

7) Laponite® XLS

Natriummagnesiumsilikat mit 6% Tetranatriumpyrophosphat

Hersteller: Laporte

8) Keltrol® F

Xanthan-gum

Hersteller: Kelco

9) Hectabrite® AW

Hectorit; Natrium/Magnesium/Lithium-Silikat; gereinigt

Hersteller: American Colloid

**Patentansprüche**

1. Wäßrige tensidhaltige Zubereitung, dadurch gekennzeichnet, daß als Tenside eine Kombination aus

    a) mindestens einem anionischen Tensid
    b) und mindestens einem Alkylpolyglykosid
    und als Verdickungsmittel eine Kombination aus
    c) einem Magnesiumaluminiumsilikat,
    d) einem wasserlöslichen, nicht-ionogenen organischen Polymer und
    e) einem Fettalkoholethoxylat
    enthalten ist.

2. Zubereitung nach Anspruch 1, dadurch gekennzeichnet, daß das Magnesiumaluminiumsilikat ein saures Magnesiumaluminiumsilikat, insbesondere vom Typ Veegum® Ultra ist.

3. Zubereitung nach Anspruch 1 oder 2, dadurch gekennzeichnet, daß das wasserlösliche, nicht-ionogene organische Polymer ausgewählt ist aus der Gruppe der Polysaccharide oder Polysaccharid-Derivate.

4. Zubereitung nach Anspruch 3, dadurch gekennzeichnet, daß das nicht-ionogene wasserlösliche Polysaccharid-Derivat eine Methylhydroxypropylcellulose ist.

5. Zubereitung nach einem der Ansprüche 1 bis 4, dadurch gekennzeichnet, daß ein Fettalkoholethoxylat mit einem HLB-Wert von 3-9 enthalten ist.

6. Zubereitung nach einem der Ansprüche 1 bis 4, dadurch gekennzeichnet, daß das Fettalkoholethoxylat eine eingeschränkte Homologenverteilung aufweist.

7. Zubereitung nach einem der Ansprüche 1 bis 6, dadurch gekennzeichnet, daß zusätzlich mindestens ein wasserlösliches Tensid ausgewählt aus der Gruppe der kationischen, zwitterionischen oder amphoteren Tenside enthalten ist.

8. Zubereitung nach einem der Ansprüche 1 bis 7, dadurch gekennzeichnet, daß ein anionisches Tensid vom Typ der Alkylethersulfate enthalten ist.

9. Zubereitung nach einem der Ansprüche 1 bis 8, dadurch gekennzeichnet, daß zusätzlich ein zwitterionisches Tensid vom Typ der Betaintenside enthalten ist.

10. Verwendung einer Zubereitung gemäß einem der Ansprüche 1-9 zur Reinigung der Haut und Haare.

Europäisches
Patentamt

# EUROPÄISCHER RECHERCHENBERICHT

Nummer der Anmeldung

EP 99 12 4450

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 97 14397 A (RECKITT & COLMANN SA ;RECKITT & COLMANN PROD LTD (GB)) 24. April 1997 (1997-04-24) * Seite 5, Zeile 4 - Zeile 14 * * Seite 9, Zeile 6 - Zeile 21 * * Seite 10, Zeile 16 - Zeile 30 * * Ansprüche * | 1,5,6,10 | A61K7/50 C11D1/83 A61K7/48 C11D1/66 A61K7/06 |
| A | EP 0 688 560 A (UNILEVER PLC ;UNILEVER NV (NL)) 27. Dezember 1995 (1995-12-27) * Seite 2, Zeile 40 - Seite 3, Zeile 10 * * Seite 4, Zeile 44 - Zeile 46 * | 1-10 | |
| A | EP 0 571 193 A (UNILEVER PLC ;UNILEVER NV (NL)) 24. November 1993 (1993-11-24) * Seite 3, Zeile 16 - Zeile 50 * * composition 2 * * Ansprüche * | 1,2,7-10 | |
| D,A | US 5 696 069 A (ITO ET. AL.) 9. Dezember 1997 (1997-12-09) * Zusammenfassung; Beispiele * | 1,2,7-10 | RECHERCHIERTE SACHGEBIETE (Int.Cl.7) |
| A | EP 0 268 064 A (HENKEL KGAA) 25. Mai 1988 (1988-05-25) * Beispiele 1,2,7 * * Seite 3, Zeile 8 - Zeile 31 * | 1-7 | A61K C11D |
| A | EP 0 612 819 A (MINNESOTA MINING & MFG) 31. August 1994 (1994-08-31) * Beispiele * | 1-4 | |
| A | EP 0 728 468 A (UNILEVER PLC ;UNILEVER NV (NL)) 28. August 1996 (1996-08-28) * Beispiel 4; Tabelle 1 * | 1,2,10 | |
| A | EP 0 832 643 A (UNILEVER PLC ;UNILEVER NV (NL)) 1. April 1998 (1998-04-01) | 1,2,10 | |

-/--

Der vorliegende Recherchenbericht wurde für alle Patentansprüche erstellt

| Recherchenort | Abschlußdatum der Recherche | Prüfer |
|---|---|---|
| DEN HAAG | 10. April 2000 | Ketterer, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
............................................................
& : Mitglied der gleichen Patentfamilie, übereinstimmendes Dokument

EPO FORM 1503 03.82 (P04C03)

**Europäisches Patentamt**

# EUROPÄISCHER RECHERCHENBERICHT

Nummer der Anmeldung

EP 99 12 4450

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | EP 0 541 347 A (UNILEVER PLC ;UNILEVER NV (NL)) 12. Mai 1993 (1993-05-12) * Seite 2, Zeile 24 - Seite 4, Zeile 12 * | 1,10 | |
| A | EP 0 339 539 A (PROCTER & GAMBLE) 2. November 1989 (1989-11-02) * Seite 6, Zeile 9 - Zeile 15 * | 1,10 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 10. April 2000 | Ketterer, M |

## ANHANG ZUM EUROPÄISCHEN RECHERCHENBERICHT
## ÜBER DIE EUROPÄISCHE PATENTANMELDUNG NR.

EP 99 12 4450

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2000

| Im Recherchenbericht angeführtes Patentdokument | | Datum der Veröffentlichung | Mitglied(er) der Patentfamilie | | | Datum der Veröffentlichung |
|---|---|---|---|---|---|---|
| WO 9714397 | A | 24-04-1997 | AU | 716159 | B | 17-02-2000 |
| | | | AU | 7224496 | A | 07-05-1997 |
| | | | BR | 9610930 | A | 21-12-1999 |
| | | | EP | 0855900 | A | 05-08-1998 |
| | | | GB | 2306323 | A,B | 07-05-1997 |
| | | | NZ | 319674 | A | 29-07-1999 |
| EP 0688560 | A | 27-12-1995 | US | 5422112 | A | 06-06-1995 |
| | | | CA | 2150788 | A | 10-12-1995 |
| | | | JP | 8053322 | A | 27-02-1996 |
| | | | ZA | 9504560 | A | 02-12-1996 |
| EP 0571193 | A | 24-11-1993 | AU | 3865593 | A | 25-11-1993 |
| | | | BR | 9302018 | A | 30-11-1993 |
| | | | CA | 2096505 | A | 22-11-1993 |
| | | | CZ | 9300966 | A | 16-03-1994 |
| | | | DE | 69326190 | D | 07-10-1999 |
| | | | DE | 69326190 | T | 23-12-1999 |
| | | | HU | 64205 | A,B | 28-12-1993 |
| | | | JP | 2566189 | B | 25-12-1996 |
| | | | JP | 6056650 | A | 01-03-1994 |
| | | | KR | 9701231 | B | 04-02-1997 |
| | | | PL | 299015 | A | 07-02-1994 |
| | | | SK | 52293 | A | 08-12-1993 |
| US 5696069 | A | 09-12-1997 | KEINE | | | |
| EP 0268064 | A | 25-05-1988 | DE | 3635535 | A | 28-04-1988 |
| | | | AT | 103321 | T | 15-04-1994 |
| | | | DE | 3789421 | D | 28-04-1994 |
| | | | ES | 2050657 | T | 01-06-1994 |
| | | | US | 4818427 | A | 04-04-1989 |
| EP 0612819 | A | 31-08-1994 | BR | 9400597 | A | 27-09-1994 |
| | | | CA | 2114356 | A | 24-08-1994 |
| | | | JP | 6256729 | A | 13-09-1994 |
| | | | MX | 9401061 | A | 31-08-1994 |
| | | | US | 5688334 | A | 18-11-1997 |
| | | | US | 5767049 | A | 16-06-1998 |
| EP 0728468 | A | 28-08-1996 | CA | 2164059 | A | 25-08-1996 |
| | | | JP | 8245368 | A | 24-09-1996 |
| EP 0832643 | A | 01-04-1998 | US | 5744148 | A | 28-04-1998 |
| | | | AU | 710602 | B | 23-09-1999 |
| | | | AU | 3416497 | A | 26-03-1998 |

Für nähere Einzelheiten zu diesem Anhang : siehe Amtsblatt des Europäischen Patentamts, Nr.12/82

## ANHANG ZUM EUROPÄISCHEN RECHERCHENBERICHT
## ÜBER DIE EUROPÄISCHE PATENTANMELDUNG NR.

EP 99 12 4450

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-04-2000

| Im Recherchenbericht angeführtes Patentdokument | | Datum der Veröffentlichung | Mitglied(er) der Patentfamilie | | Datum der Veröffentlichung |
|---|---|---|---|---|---|
| EP 0832643 | A | | BR | 9704766 A | 29-12-1998 |
| | | | CA | 2213036 A | 20-03-1998 |
| | | | CN | 1185942 A | 01-07-1998 |
| | | | JP | 10101525 A | 21-04-1998 |
| EP 0541347 | A | 12-05-1993 | AU | 667843 B | 18-04-1996 |
| | | | AU | 2818792 A | 13-05-1993 |
| | | | CA | 2081932 A | 08-05-1993 |
| | | | JP | 6192036 A | 12-07-1994 |
| | | | ZA | 9208575 A | 06-05-1994 |
| EP 0339539 | A | 02-11-1989 | AU | 635205 B | 18-03-1993 |
| | | | AU | 3375989 A | 02-11-1989 |
| | | | CA | 1328816 A | 26-04-1994 |
| | | | CN | 1037271 A | 22-11-1989 |
| | | | DK | 207289 A | 29-10-1989 |
| | | | FI | 892014 A | 29-10-1989 |
| | | | JP | 2056412 A | 26-02-1990 |
| | | | NZ | 228466 A | 28-05-1990 |
| | | | PH | 25172 A | 27-03-1991 |
| | | | US | 4854333 A | 08-08-1989 |

EPO FORM P0461

Für nähere Einzelheiten zu diesem Anhang : siehe Amtsblatt des Europäischen Patentamts, Nr.12/82